# EUROPEAN PATENT APPLICATION

(11) **EP 4 043 577 A1**
(43) Date of publication of application: **17.08.2022**
(21) Application number: 21156519.7
(22) Date of filing: 11.02.2021
(51) Int. Cl.: C12P 7/02, C12P 7/62, C12P 41/00

(54) **ENANTIOSELECTIVE HYDROLYSIS**

(71) Applicant: Technische Universität Graz, 8010 Graz (AT)
(72) Inventor: KOURIST, Robert, 8010 Graz (AT); CALDERINI, Elia, 8010 Graz (AT); DRIENOVSKA, Ivana, 8010 Graz (AT); SCHWAB, Helmut, 8010 Graz (AT); HOFER, Michael, 94315 Straubing (DE)
(74) Representative: Schwarz & Partner Patentanwälte GmbH

(57) **Abstract**

The present invention relates to a method of hydrolysing bicyclic monoterpenes with α/β-hydrolase-fold esterases.

SEQUENCE LISTING

<110>  Technische Universität Graz

<120>  ENANTIOSELECTIVE HYDROLYSIS

<130>  27419-EP

<160>  5

<170>  PatentIn version 3.5

<210>  1
<211>  392
<212>  PRT
<213>  Burkholderia gladioli

<400>  1

Met Thr Ala Ala Ser Leu Asp Pro Thr Ala Phe Ser Leu Asp Ala Ala
1               5                   10                  15

Ser Leu Ala Ala Arg Leu Asp Ala Val Phe Asp Gln Ala Leu Arg Glu
            20                  25                  30

Arg Arg Leu Val Gly Ala Val Ala Ile Val Ala Arg His Gly Glu Ile
        35                  40                  45

Leu Tyr Arg Arg Ala Gln Gly Leu Ala Asp Arg Glu Ala Gly Arg Pro
    50                  55                  60

Met Arg Glu Asp Thr Leu Phe Arg Leu Ala Ser Val Thr Lys Pro Ile
65                  70                  75                  80

Val Ala Leu Ala Val Leu Arg Leu Val Ala Arg Gly Glu Leu Ala Leu
                85                  90                  95

Asp Ala Pro Val Thr Arg Trp Leu Pro Glu Phe Arg Pro Arg Leu Ala
            100                 105                 110

Asp Gly Ser Glu Pro Leu Val Thr Ile His His Leu Leu Thr His Thr
        115                 120                 125

Ser Gly Leu Gly Tyr Trp Leu Leu Glu Gly Ala Gly Ser Val Tyr Asp
    130                 135                 140

Arg Leu Gly Ile Ser Asp Gly Ile Asp Leu Arg Asp Phe Asp Leu Asp
145                 150                 155                 160

Glu Asn Leu Arg Arg Leu Ala Ser Ala Pro Leu Ser Phe Ala Pro Gly
                165                 170                 175

Ser Gly Trp Gln Tyr Ser Leu Ala Leu Asp Val Leu Gly Ala Val Val
            180                 185                 190

Glu Arg Ala Thr Gly Gln Pro Leu Ala Ala Ala Val Asp Ala Leu Val
        195                 200                 205

Ala Gln Pro Leu Gly Met Arg Asp Cys Gly Phe Val Ser Ala Glu Pro
    210                 215                 220

Glu Arg Phe Ala Val Pro Tyr His Asp Gly Gln Pro Glu Pro Val Arg
225                 230                 235                 240

Met Arg Asp Gly Ile Glu Val Pro Leu Pro Glu Gly His Gly Ala Ala
                245                 250                 255

Val Arg Phe Ala Pro Ser Arg Val Phe Glu Pro Gly Ala Tyr Pro Ser
            260                 265                 270

Gly Gly Ala Gly Met Tyr Gly Ser Ala Asp Asp Val Leu Arg Ala Leu
        275                 280                 285

Glu Ala Ile Arg Ala Asn Pro Gly Phe Leu Pro Glu Thr Leu Ala Asp
    290                 295                 300

Ala Ala Arg Arg Asp Gln Ala Gly Val Gly Ala Glu Thr Arg Gly Pro
305                 310                 315                 320

Gly Trp Gly Phe Gly Tyr Leu Ser Ala Val Leu Asp Asp Pro Ala Ala
                325                 330                 335

Ala Gly Thr Pro Gln His Ala Gly Thr Leu Gln Trp Gly Gly Val Tyr
            340                 345                 350

Gly His Ser Trp Phe Val Asp Arg Ala Leu Gly Leu Ser Val Leu Leu
        355                 360                 365

Leu Thr Asn Thr Ala Tyr Glu Gly Met Ser Gly Pro Leu Thr Ile Ala
    370                 375                 380

Leu Arg Asp Ala Val Tyr Ala Arg
385                 390

<210> 2
<211> 4
<212> PRT
<213> Artificial Sequence

<220>
<223> Catalytic motif

<220>
<221>  MISC_FEATURE
<222>  (2)..(3)
<223>  X is any amino acid

<400>  2

Ser Xaa Xaa Lys
1

<210>  3
<211>  392
<212>  PRT
<213>  Artificial Sequence

<220>
<223>  NK70 mutant of esterase B of B. gladioli

<400>  3

Met Thr Ala Ala Ser Leu Asp Pro Thr Ala Phe Ser Leu Asp Ala Ala
1               5                   10                  15

Leu Leu Ala Ala Arg Leu Asp Ala Val Phe Asp Gln Ala Leu Arg Glu
            20                  25                  30

Arg Arg Leu Val Gly Ala Val Ala Ile Val Ala Arg His Gly Glu Ile
        35                  40                  45

Leu Tyr Arg Arg Ala Gln Gly Leu Ala Asp Arg Glu Ala Gly Arg Pro
    50                  55                  60

Met Arg Glu Asp Thr Leu Phe Arg Leu Ala Ser Val Thr Lys Pro Ile
65                  70                  75                  80

Val Ala Leu Ala Val Leu Arg Leu Val Ala Arg Gly Glu Leu Ala Leu
                85                  90                  95

Asp Ala Pro Val Thr Arg Trp Leu Pro Glu Phe Arg Pro Arg Leu Ala
            100                 105                 110

Asp Gly Ser Glu Pro Leu Val Thr Ile His His Leu Leu Thr His Thr
        115                 120                 125

Ser Gly Leu Ser Tyr Trp Leu Leu Glu Gly Ala Gly Ser Val Tyr Asp
    130                 135                 140

Arg Leu Gly Ile Ser Asp Gly Ile Asp Leu Arg Asp Phe Asp Leu Asp
145                 150                 155                 160

Glu Asn Leu Arg Arg Leu Ala Ser Ala Pro Leu Ser Phe Ala Pro Gly
                165                 170                 175

Ser Gly Trp Gln Tyr Ser Leu Ala Leu Asp Val Leu Gly Ala Val Val
            180                 185                 190

Glu Arg Ala Thr Gly Gln Pro Leu Ala Ala Ala Val Asp Ala Leu Val
        195                 200                 205

Ala Gln Pro Leu Gly Met Arg Asp Cys Gly Phe Val Ser Ala Glu Pro
    210                 215                 220

Glu Arg Phe Ala Val Pro Tyr His Asp Gly Gln Pro Glu Pro Val Arg
225                 230                 235                 240

Met Arg Asp Gly Ile Glu Val Pro Leu Pro Gly Gly His Gly Ala Ala
                245                 250                 255

Val Arg Phe Ala Pro Ser Arg Val Phe Glu Pro Gly Ala Tyr Pro Ser
            260                 265                 270

Gly Gly Ala Gly Met Tyr Gly Ser Ala Asp Asp Val Leu Arg Ala Leu
        275                 280                 285

Glu Ala Ile Arg Ala Asn Pro Gly Phe Leu Pro Glu Thr Leu Ala Asp
    290                 295                 300

Ala Ala Arg Arg Asp Gln Val Gly Val Gly Ala Lys Thr Arg Gly Pro
305                 310                 315                 320

Gly Trp Gly Phe Gly Tyr Leu Ser Ala Val Leu Asp Asp Pro Ala Ala
                325                 330                 335

Ala Gly Thr Pro Gln His Ala Gly Thr Leu Gln Trp Gly Gly Val Tyr
            340                 345                 350

Gly His Ser Trp Phe Val Asp Arg Ala Leu Gly Leu Ser Val Leu Leu
        355                 360                 365

Leu Thr Asn Thr Ala Tyr Glu Gly Met Ser Gly Pro Leu Thr Ile Ala
    370                 375                 380

Leu Arg Asp Ala Val Tyr Ala Arg
385                 390

<210>  4
<211>  493
<212>  PRT

<213>  Rhodococcus rhodochrous

<400>  4

Met Glu Pro Val Val Asp Thr Thr Gly Gly Arg Val Arg Gly Thr Thr
1               5                   10                  15

Val Gly Gly Thr Ala Val Phe Lys Gly Ile Pro Tyr Ala Ala Ala Pro
            20                  25                  30

Phe Gly Glu Leu Arg Phe Ala Ala Pro Gly Pro Val Pro Arg Trp Asp
        35                  40                  45

Gly Val Arg Pro Ala Thr Glu Phe Gly Pro Thr Ala Pro Lys Ala Pro
    50                  55                  60

Tyr Ala Pro Pro Ile Asp Gln Leu Leu His Glu Pro Ser Ile Pro Gly
65                  70                  75                  80

Glu Asp Val Leu Gly Leu Asn Val Trp Ser Pro Asp Leu Ser Gly Ser
                85                  90                  95

Leu Pro Val Ile Val Trp Ile His Gly Gly Ala Phe Val His Gly Ser
            100                 105                 110

Ser Ala Val Ala Leu Tyr Asp Gly Ala Asn Phe Ala Arg Asp Gly Val
        115                 120                 125

Val Phe Leu Ser Ile Asn Tyr Arg Leu Gly Ala Glu Gly Phe Ala Phe
    130                 135                 140

Leu Asp Asp Ala Pro Ala Asn Arg Gly Leu Leu Asp Gln Val Ala Ala
145                 150                 155                 160

Leu Ala Trp Val Arg Asp Asn Ile Ala Ala Phe Gly Gly Asp Pro Asp
                165                 170                 175

Arg Val Thr Ile Ala Gly Glu Ser Ala Gly Ala Met Ser Val Val Ser
            180                 185                 190

Leu Leu Ala Met Pro Ala Ala Arg Gly Leu Phe Arg Arg Ala Ile Ala
        195                 200                 205

Gln Ser Gly Ala Gly His Ser Val Met Ser Ala Gly Thr Ala Arg Thr
    210                 215                 220

Val Ala Gly Ala Leu Gly Asp Met Leu Gly Val Ala Pro Thr Arg Ala
225                 230                 235                 240

Ala Phe Ala Glu Ile Asp Pro Lys Val Leu Val Asp Ala Gln Glu Arg
                245                 250                 255

Leu Thr Ala Arg Ile Arg Gly Glu Arg Asp Pro Ala Thr Trp Gly Glu
            260                 265                 270

Ile Ala Thr Asp Ser Met Ala Phe Glu Pro Cys Val Asp Gly Thr Val
        275                 280                 285

Leu Pro Asp Arg Pro Ile Asp Leu Val Ala Asn Gly Ala Gly Ser Asp
    290                 295                 300

Val Asp Val Leu Ile Gly His Asn Gly Asp Glu Met Thr Leu Phe Phe
305                 310                 315                 320

Val Pro Phe Gly Val Asp Gly Leu Leu Asp Glu Val Ala Ala Arg Gly
                325                 330                 335

Leu Val Ala Ala Tyr Gly Leu Thr Asp Pro Glu Ala Phe Asp Ala Tyr
            340                 345                 350

Arg Arg Ala Arg Pro Asp Ala Arg Pro Gly Asp Thr Ala Met His Ile
        355                 360                 365

Val Arg Asp Trp Met Tyr Arg Ile Pro Val Leu Arg Val Ala Glu Ala
    370                 375                 380

Arg Ala Thr His Gly Ala Glu Thr His Val Tyr Arg Phe Asp Trp Lys
385                 390                 395                 400

Ser Pro Leu Phe Asp Gly Arg Leu Gly Ala Thr His Ala Leu Asp Ile
                405                 410                 415

Gly Phe Val Phe Asp Asn Leu His Leu Pro Gln Ala Ala Ala Leu Ala
            420                 425                 430

Gly Ser Ala Pro Pro Gln Glu Leu Ala Thr Arg Met His Arg Ala Trp
        435                 440                 445

Val Asp Phe Ala Thr Thr Gly Ser Pro Gly Trp Pro Pro Tyr Gly Asp
    450                 455                 460

Glu Arg Thr Glu Met Leu Phe Gly Asp Thr Cys Gly Thr Val Asp Asp
465                 470                 475                 480

Ala Glu Ala Glu Leu Arg Thr Leu Trp Asp Gly Ile Arg
                485                 490

<210>  5
<211>  5
<212>  PRT
<213>  Artificial Seqence

<220>
<223>  Catalytic motif



<220>
<221>  MISC_FEATURE
<222>  (2)..(2)
<223>  X is a chain of amino acids of a length of 20 to 200 amino acids

<220>
<221>  MISC_FEATURE
<222>  (4)..(4)
<223>  X is a chain of amino acids of a length of 20 to 200 amino acids

<400>  5

Ser Xaa Glu Xaa His
1               5

## Description

### TECHNICAL FIELD

The present invention relates to the field of enantioselective hydrolysis.

### BACKGROUND ART

The demand for enantiopure compounds and synthetic pathways to access single enantiomers is constantly increasing in the chemical and pharmaceutical industry, e.g. due to stricter requirements by the national regulatory agencies. This is in particular the case for monoterpenes like bicyclic monoterpenes.

Monoterpenes can accumulate as side-products during processes such as the cellulose production from conifers and thus are considered promising feedstocks for the synthesis of chemical products from renewable resources as a substitute for petrol-based chemicals.

In particular, monoterpenoids such as isoborneol, borneol and camphor, but also fenchols, find application in many products such as food flavouring, cosmetics and cleaning products. Diverse biological activities such as anti-inflammatory, vasorelaxant and neuroprotective, among others, make them also valuable ingredients for health-related formulations.

The monoterpene camphor and its corresponding alcohols, borneol and isoborneol, find application as fragrances and in traditional Chinese medicine. The pure enantiomers of isoborneol and borneol are frequently found in essential oils from many plants, and they have shown a wide array of biological and antimicrobial activities. Derivatives of these monoterpenoids find also application as chiral ligands in asymmetric synthesis.

(+)-camphor (or (1R)-camphor), for instance, can be isolated from various natural sources and is widely applied in cosmetics and natural medicine, whereas racemic camphor can be obtained from α-pinene, a side-product from turpentine production. Isomerization of α-pinene yields camphene and afterwards, the addition of organic acids to camphene leads to isomerization and the formation of rac-isobornyl esters. Finally, hydrolysis and oxidation yield rac-camphor.

Chemical reduction of rac-camphor produces a mixture of racemic borneol and isoborneol, which is called "synthetic borneol". Reduction of (+)-camphor leads to a mixture of (+)-borneol and (-)-isoborneol. As the separation of the isomers is costly, they are usually marketed as a mixture under the assignation "semisynthetic borneol". Pure (1R)-borneol can be extracted from the resin and essential oil of woody plants of the families Dipterocarpaceae, Lamiaceae, Valerianaceae and Asteraceae. Pure (-)-borneol can be isolated from the herbaceous plant Blumea balsamifera. While (+)-camphor and rac-camphor are easily available, (-)-camphor is more difficult to obtain, as it is usually produced via oxidation of (-)-borneol, which is however only available by extraction from plants. Therefore, known routes for the synthesis of (-)-camphor are cumbersome and costly.

Alternatively, (-)-borneol is known to be available by kinetic resolution of racemic borneol using dehydrogenases. Enantioselective borneol-dehydrogenases from Salvia officinalis L have been described (I. Drienovská et al., Phytochemistry (2020): 172, 112227). These enzymes show high enantioselectivity in the kinetic resolution of borneol and isoborneol and require cofactors. Thus, the necessity to recycle the NAD+-cofactor represents a major disadvantage when using borneol-dehydrogenases.

Consequently, it is an object of the present invention to provide a simple and reliable method for obtaining enantiopure monoterpenes such as (-)-camphor.

### SUMMARY OF THE INVENTION

The present invention relates to a method for hydrolysing a compound, a bicyclic monoterpene, of general formula (I) 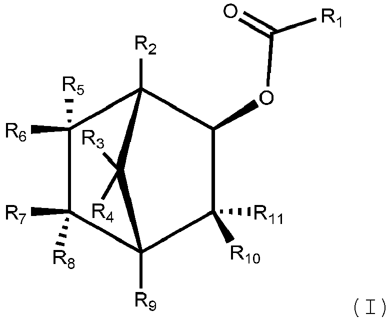 or a compound of general formula (II) 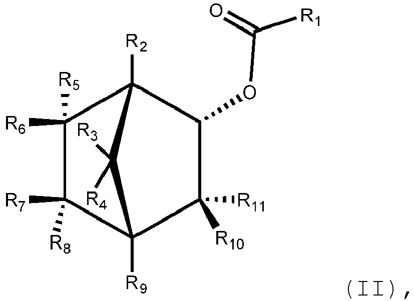 wherein R₁, R₂, R₃ and R₄ are independently from each other a C₁ to C₅ alkyl group or hydrogen, and R₅, R₆, R₇, R₈, R₉, R₁₀ and R₁₁ are independently from each other a C₁ or C₂ alkyl group or hydrogen,
said method comprising the step of incubating at least one compound of general formula (I) or (II) with an α/β-hydrolase-fold esterase.

Surprisingly, it turned out that the ester group of a compound (i.e. of a bicyclic monoterpene) of general formula (I) and (II) is converted to an alcohol group by using an α/β-hydrolase-fold esterase with high selectivity. This is particularly surprising since the ester group in the compound of general formula (I) or (II) is attached to a bulky residue (i.e. the backbone of the bicyclic monoterpene) and is therefore sterically hindered.

In a preferred embodiment of the present invention, the compound of general formula (I) or (II) is comprised in a composition further comprising a compound of general formula (I') 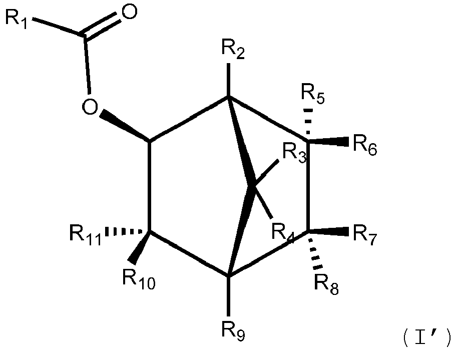 or a compound of general formula (II')

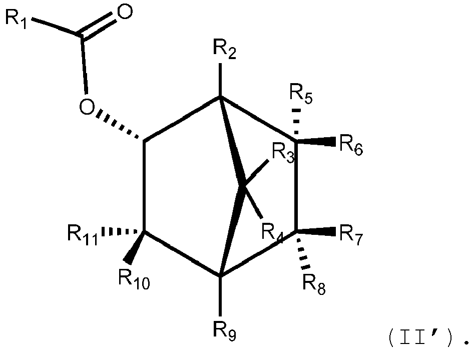

The method according to the present invention allows the selective hydrolysis of a compound of general formula (I) or of general formula (II) in a composition comprising a compound of general formula (I) and a compound of general formula (I'), or of general formula (II) and a compound of general formula (II'), respectively.

Surprisingly, α/β-hydrolase-fold esterases are particularly suitable for the kinetic resolution of racemic compounds of general formula (I)/(I') or of racemic compounds of general formula (II)/(II').

The use of an α/β-hydrolase-fold esterase allows separating the enantiomers of general formula (I) and (I'). The α/β-hydrolase-fold esterase selectively catalyzes the hydrolysis of the compound of general formula (I), while the compound of general formula (I') is not converted, or converted to a significantly lower extent, as reflected by high values of the enantiomeric excess (ee) and enantioselectivity (E).

For example, the method according to the present invention allows for the enantioselective hydrolysis of racemic isobornyl esters to selectively obtain (+)-isoborneol ((1S,2S,4S)-isoborneol). Similarly, (+)-endofenchol ((1R,2R,4S)-endofenchol) can be obtained from racemic endofenchylesters. In these cases, the α/β-hydrolase-fold esterase may be a carboxyl esterase comprising or consisting of an amino acid sequence with at least 80 % identity to SEQ ID No. 1 or 3.

The method according to the present invention for example also allows for the enantioselective hydrolysis of racemic bornyl esters to selectively obtain (-)-borneol ((1S,2R,4S)-borneol). In this case, the α/β-hydrolase-fold esterase may be a carboxyl esterase comprising or consisting of an amino acid sequence with at least 80 % identity to SEQ ID No. 4.

From enantiopure (+)-isoborneol ((1S,2S,4S)-isoborneol) and enantiopure (-)-borneol ((1S,2R,4S)-borneol), (-)-camphor ((1S)-camphor) is easily accessible by oxidation.

### DESCRIPTION OF EMBODIMENTS

The present invention relates to the enzymatic hydrolysis of a compound of general formula (I) or (II) by incubating said compound with an α/β-hydrolase-fold esterase. The method according to the present invention is particularly advantageous to resolve a racemic mixture of a compound of general formula (I) and the enantiomer thereof, i.e. a compound of general formula (I'), or of general formula (II) and the enantiomer thereof, i.e. a compound of general formula (II').

The compounds of general formula (I) or (II) represent carboxylic esters of chiral bicyclic monoterpenes, which are derivatives of norbornane (bicyclo[2.2.1]heptan). Preferably, the compounds of general formula (I) or (II) are bornanes or derivatives thereof. The carboxylic ester group in the compound of general formula (I) is in exo position with respect to the bridged ring system. In contrast, the carboxylic ester group in the compound of general formula (II) is in endo position with respect to the bridged ring system.

According to the present invention, the substituents R₁, R₂, R₃ and R₄ are independently from each other a C₁ to C₅ alkyl group or hydrogen. Preferably, R₁, R₂, R₃ and R₄ are independently from each other a C₁ to C₃ alkyl group or hydrogen. The substituents R₅, R₆, R₇, R₈, R₉, R₁₀ and R₁₁ are independently from each other a C₁ or C₂ alkyl group or hydrogen.

As used herein, the terms "C₁ to C₅ alkyl group" and "C₁ to C₃ alkyl group" refer to alkyl radicals having 1 to 5 and 1 to 3 carbon atoms, respectively, which may be branched or arranged linearly. Preferably, the carbon atoms are arranged linearly (i.e., a linear alkyl radical). Accordingly, a "C₁ to C₂ alkyl group" comprises alkyl radicals having 1 to 2 carbon atoms.

The alkyl groups according to the present invention may comprise one or more further substituents, such as a functional group selected from the group consisting of an ester group, an ether group, a sulfonyl group, a nitro group and derivatives thereof.

In a preferred embodiment, R₁ in the compound of general formula (I) and (I') or (II) and (II') is a C₁ alkyl group (methyl group) or a C₃ alkyl group (propyl group). Thus, the compound of general formula (I) and (I') or (II) and (II') is preferably an acetate or a butyrate.

In one preferred embodiment, R₂, R₃, R₄, R₁₀ and/or R₁₁ is a methyl group or hydrogen, and R₅, R₆, R₇, R₈ and R₉ is hydrogen.

According to a preferred embodiment, each of R₂, R₃ and R₄ is a methyl group and each of R₅, R₆, R₇, R₈, R₉, R₁₀ and R₁₁ is hydrogen. Thus, the compound of general formula (I) and (I') is preferably an isobornylester, and the compound of general formula (II) and (II') is preferably a bornylester. The ester is preferably an acetate or a butyrate.

According to another preferred embodiment, each of R₂, R₁₀ and R₁₁ is a methyl group, and each of R₃, R₄, R₅, R₆, R₇, R₈ and R₉ is hydrogen. Thus, the compound of general formula (I) and (I') is preferably an exofenchylester and the compound of general formula (II) and (II') is preferably an endofenchylester.

It has been found that the method according to the present invention is particularly suitable to hydrolyse isobornylesters, bornylesters and endofenchylesters.

The enzyme according to the present invention is an α/β-hydrolase-fold esterase, preferably a carboxyl esterase.

The term "α/β hydrolase" summarizes a group of hydrolytic enzymes, which are characterized by the α/β hydrolase folding in the enzyme core, as described by Ollis et al., Protein Engineering 1992; 5(3): 197-211. The term "carboxyl esterase" (also referred to as "carboxylic-ester hydrolase") refers to an enzyme, which catalyzes the hydrolysis of a carboxylic ester to an alcohol and a carboxylate.

As used herein, an "α/β-hydrolase-fold esterase" is an esterase characterized by the α/β hydrolase folding in the enzyme core, which esterase catalyzes the hydrolysis of a compound of general formula (I) or (II) as defined herein. The α/β-hydrolase-fold esterase according to the present invention can be isolated from its original organism, or can be obtained by recombinant expression or chemical synthesis.

Surprisingly, α/β-hydrolase-fold esterases have been found to be particularly suitable for hydrolysing a compound of general formula (I) or (II) as defined herein.

According to the present invention, at least one compound of general formula (I) or (II) is incubated with an α/β-hydrolase-fold esterase. The term "incubated" as used herein is to be understood as bringing at least one compound of general formula (I) or (II) into physical contact with the α/β-hydrolase-fold esterase. Preferably, incubation is performed in the liquid phase.

According to a preferred embodiment, incubation is performed in solution at a pH of 4 to 9, preferably, 7 to 8, and at a temperature of 10 to 60 °C, preferably 20 to 30 °C. Preferably, the sample is stirred to improve the contact of the compound of general formula (I) or (II) with the α/β-hydrolase-fold esterase. As a solvent, an aqueous buffer solution is preferred, e.g. an aqueous phosphate buffer such as potassium phosphate buffer at pH 7. Optionally, a cosolvent such as dimethyl sulfoxide (DMSO) or N,N-dimethylformamide (DMF) may be used to increase the solubility. The compound of general formula (I) or (II) may be applied at a concentration of 0,05 mM to 100 mM, more preferably 0,1 mM to 10 mM, even more preferably 0,5 to 5 mM, most preferably 0,8 to 1,5 mM, e.g. 1 mM. The α/β-hydrolase-fold esterase may for example be applied as a cell-free extract with a total protein content in the range of 0,5 g/L to 50 g/L, preferably 1 g/L to 20 g/L, even more preferably 5 g/L to 15 g/L, e.g. 8 to 12 g/L. In preferred embodiments, around 5 to 30 wt% of the total protein content in the cell-free extract may correspond to the α/β-hydrolase-fold esterase. Suitable concentrations of the compound of general formula (I) or (II) and of the α/β-hydrolase-fold esterase are concentrations at which sufficient dissolution and sufficient reactivity can be obtained. According to one embodiment of the present invention, the compound of general formula (I) or (II) is comprised in a composition further comprising a compound of general formula (I') or (II').

Preferably, said composition comprises the compound of general formula (I) and the compound of general formula (I'), or the compound of general formula (II) and the compound of general formula (II').

According to a particularly preferred embodiment, the composition is a racemic composition.

According to one aspect of the present invention, said α/β-hydrolase-fold esterase is a carboxyl esterase comprising or consisting of an amino acid sequence with at least 80 %, preferably at least 85%, more preferably at least 90 %, even more preferably at least 95 %, even more preferably at least 98%, even more preferably at least 99%, identity to SEQ ID No. 1, wherein said α/β-hydrolase-fold esterase comprises the catalytic motif of Ser-X-X-Lys (SEQ ID No. 2), wherein X is any amino acid residue. In SEQ ID NO.2, "X-X" may be any sequence of two amino acid residues, wherein the two amino acid residues may be different or identical. Preferably, one of the amino acid residues is hydrophobic and one of the amino acid residues is hydrophilic. For example, "X-X" may be "V-T", or any other combination of two amino acid residues.

"Identity", as used herein, means that two or more amino acid sequences can have a certain "identity" (matching amino acid residues at identical positions) to each other when they are stored on top of each other. Herein, "identity" is defined as the percentage number of amino acids of the candidate amino acid sequences that are identical to the amino acids of the original sequence, after matching the two sequences and introducing gaps, if necessary, to achieve the maximum percentage sequence identity as generated by the program "Protein BLAST" (blastp; Altschul et al., J. Mol. Biol. (1997) 215:403-410; http://blast.ncbi.nlm.nih.gov/Blast.cgi; here generally referred to as "BLAST"). Herein, the algorithm "blastp (protein-protein BLAST)" is used with the following parameters: expected threshold: 0.05; word size: 6; matrix: BLOSUM62; gap costs: existence 11, extension 1; conditional compositional score matrix adjustment; no filter and no mask.

A percentage (%) value for amino acid sequence identity is defined by the number of matching identical nucleotides divided by the sequence length, for which the percentage identity is determined.

In a specific embodiment, said carboxyl esterase is wild type Esterase B of Burkholderia gladioli: 
SEQ ID No. 1, UniProt accession number: Q9KX40; amino acid
sequence:

MTAASLDPTA FSLDAASLAA RLDAVFDQAL RERRLVGAVA IVARHGEILY
RRAQGLADRE AGRPMREDTL FRLASVTKPI VALAVLRLVA RGELALDAPV
TRWLPEFRPR LADGSEPLVT IHHLLTHTSG LGYWLLEGAG SVYDRLGISD
GIDLRDFDLD ENLRRLASAP LSFAPGSGWQ YSLALDVLGA VVERATGQPL
AAAVDALVAQ PLGMRDCGFV SAEPERFAVP YHDGQPEPVR MRDGIEVPLP
EGHGAAVRFA PSRVFEPGAY PSGGAGMYGS ADDVLRALEA IRANPGFLPE
TLADAARRDQ AGVGAETRGP GWGFGYLSAV LDDPAAAGTP QHAGTLQWGG
VYGHSWFVDR ALGLSVLLLT NTAYEGMSGP LTIALRDAVY AR,
 or the NK70 mutant thereof:

SEQ ID No. 3, amino acid sequence:

MTAASLDPTA FSLDAALLAA RLDAVFDQAL RERRLVGAVA IVARHGEILY
RRAQGLADRE AGRPMREDTL FRLASVTKPI VALAVLRLVA RGELALDAPV
TRWLPEFRPR LADGSEPLVT IHHLLTHTSG LSYWLLEGAG SVYDRLGISD
GIDLRDFDLD ENLRRLASAP LSFAPGSGWQ YSLALDVLGA VVERATGQPL
AAAVDALVAQ PLGMRDCGFV SAEPERFAVP YHDGQPEPVR MRDGIEVPLP
GGHGAAVRFA PSRVFEPGAY PSGGAGMYGS ADDVLRALEA IRANPGFLPE
TLADAARRDQ VGVGAKTRGP GWGFGYLSAV LDDPAAAGTP QHAGTLQWGG
VYGHSWFVDR ALGLSVLLLT NTAYEGMSGP LTIALRDAVY AR.

This NK70 mutant comprises the amino acid substitutions S17L, G132S, E251G, A311V and E316K.

Using a carboxyl esterase comprising or consisting of an amino acid sequence with at least 80 % identity to SEQ ID No. 1 as the α/β-hydrolase-fold esterase according to the present invention allows to perform the hydrolysis of a compound of general formula (I) or (II) at high conversion and high selectivity.

In one embodiment, the compound of general formula (I) or (II) is hydrolysed if the α/β-hydrolase-fold esterase is a carboxyl esterase comprising or consisting of an amino acid sequence with at least 80%, preferably at least 85%, more preferably at least 90%, even more preferably at least 95%, even more preferably at least 98%, even more preferably at least 99%, identity to SEQ ID No. 1, wherein said α/β-hydrolase-fold esterase comprises the catalytic motif of Ser-X-X-Lys (SEQ ID No. 2), wherein X is any amino acid residue. Preferably, the compound of general formula (I) or (II) is hydrolysed if the α/β-hydrolase-fold esterase is wild type Esterase B from Burkholderia gladioli (SEQ ID No. 1) or the NK70 mutant thereof (SEQ ID No. 3). According to one embodiment of the inventive method, the composition described herein comprises the compound of general formula (I) and the compound of general formula (I'), or the compound of general formula (II) and the compound of general formula (II'), wherein said composition is preferably a racemic composition, and the α/β-hydrolase-fold esterase is a carboxyl esterase comprising or consisting of an amino acid sequence with at least 80%, preferably at least 85%, more preferably at least 90%, even more preferably at least 95%, even more preferably at least 98%, even more preferably at least 99% identity to SEQ ID No. 1, wherein said α/β-hydrolase-fold esterase comprises the catalytic motif of Ser-X-X-Lys (SEQ ID No. 2), wherein X is any amino acid residue. Preferably, said carboxyl esterase has an amino acid sequence of SEQ ID No. 1 or SEQ ID No. 3.

Preferably, the compound of general formula (I) or (II) is hydrolysed to the corresponding alcohol in said composition comprising a compound of general formula (I) and (I'), or of general formula (II) and (II'), respectively, in an enantiomeric excess of at least 80 %, preferably at least 90 %, even more preferably at least 91 %. According to the present invention, the enantiomeric excess value is preferably obtained from chiral gas chromatography.

Thus, the inventive method is particularly advantageous to separate enantiomers of general formula (I) and (I'), or to separate enantiomers of general formula (II) and (II').

According to a preferred embodiment of the present invention the compound of general formula (I) is a (1S,2S,4S)-isobornylester ((+)-isobornylester) and the compound of general formula (I') is a (1R,2R,4R)-isobornylester ((-)-isobornylester), and said (1S,2S,4S)-isobornylester is hydrolysed to (1S,2S,4S)-isoborneol. Preferably, said (1S,2S,4S)-isobornylester is (1S,2S,4S)-isobornylbutyrate and said (1R,2R,4R)-isobornylester is (1R,2R,4R)-isobornylbutyrate. It has been found that a particularly high conversion and selectivity is obtained if said (1S,2S,4S)-isobornylester is (1S,2S,4S)-isobornylbutyrate and said (1R,2R,4R)-isobornylester is (1R,2R,4R)-isobornylbutyrate.

Said (1S,2S,4S)-isoborneol may be further converted to (1S)-camphor. (1S)-camphor can be obtained by oxidizing (1S,2S,4S)-isoborneol. Oxidation to (1S)-camphor can be easily performed for example by mixing Oxone and catalytic amounts of sodium chloride at room temperature, as described by P.T. Lang et al., J. Chem. Educ. 88 (2011): 652-656.

Racemic camphor is typically synthesized by converting (isomerizing) α-pinene to camphene, which is converted to racemic isobornyl ester, which isobornyl ester is in turn hydrolysed to obtain racemic isoborneol. From racemic isoborneol, racemic camphor is obtained via chemical oxidation. The method according to the present invention may substitute the unselective hydrolytic step from isobornyl ester to racemic isoborneol in the in existing synthetic route from α-pinene to camphor. Thereby, a facile procedure for the manufacture of pure (1S,2S,4S)-isoborneol and (1R,2R,4R)-(-)-isobornyl butyrate is provided. This is particularly advantageous, since pure (1S) camphor, which is a precious and desired product, is easily obtainable from pure (1S,2S,4S)-isoborneol by oxidation, for example by using an oxidation method as described above.

According to a preferred embodiment, the compound of general formula (II) is a (1R,2R,4S)-endofenchylester ((+)-endofenylester) and said compound of general formula (II') is a (1S,2S,4R)-endofenchylester ((-)-endofenylester), and said (1R,2R,4S)-endofenchylester is hydrolysed to (1R,2R,4S)-endofenchol. Preferably, said (1R,2R,4S)-endofenchylester is (1R,2R,4S)-endofenchylbutyrate and said (1S,2S,4R)-endofenchylester is (1S,2S,4R)-endofenchylbutyrate.

According to another aspect of the present invention, said α/β-hydrolase-fold esterase is a carboxyl esterase comprising or consisting of an amino acid sequence with at least 80%, preferably at least 85%, more preferably at least 90%, even more preferably at least 95%, even more preferably at least 98%, even more preferably at least 99%, identity to SEQ ID No. 4, wherein said α/β-hydrolase-fold esterase comprises the catalytic motif of Ser-(Z)ₙ-Glu-(Z)ₘ-His (SEQ ID No. 5), wherein (Z)ₙ and (Z)ₘ are a chain of amino acids of a length of 20 to 200 amino acids (Z is any amino acid residue; n is between 20 and 200 and m is between 20 and 200).

In a specific embodiment, said carboxyl esterase is Esterase C from Rhodococcus rhodochrous:

SEQ ID No. 4, amino acid sequence:

MEPVVDTTGG RVRGTTVGGT AVFKGIPYAA APFGELRFAA PGPVPRWDGV
RPATEFGPTA PKAPYAPPID QLLHEPSIPG EDVLGLNVWS PDLSGSLPVI
VWIHGGAFVH GSSAVALYDG ANFARDGVVF LSINYRLGAE GFAFLDDAPA
NRGLLDQVAA LAWVRDNIAA FGGDPDRVTI AGESAGAMSV VSLLAMPAAR
GLFRRAIAQS GAGHSVMSAG TARTVAGALG DMLGVAPTRA AFAEIDPKVL
VDAQERLTAR IRGERDPATW GEIATDSMAF EPCVDGTVLP DRPIDLVANG
AGSDVDVLIG HNGDEMTLFF VPFGVDGLLD EVAARGLVAA YGLTDPEAFD
AYRRARPDAR PGDTAMHIVR DWMYRIPVLR VAEARATHGA ETHVYRFDWK
SPLFDGRLGA THALDIGFVF DNLHLPQAAA LAGSAPPQEL ATRMHRAWVD
FATTGSPGWP PYGDERTEML FGDTCGTVDD AEAELRTLWD GIR.

Esterase C from Rhodococcus rhodochrous (SEQ. ID No. 4) comprises the catalytic motif of Ser-(Z)ₙ-Glu-(Z)ₘ-His (SEQ ID No. 5), wherein Ser is at position 184, Glu is at position 315 and His is at position 412. Thus, in this case, (Z)ₙ is a chain of 131 amino acids and (Z)ₘ is a chain of 97 amino acids.

Using a carboxyl esterase comprising or consisting of an amino acid sequence with at least 80 % identity to SEQ ID No. 4 as the α/β-hydrolase-fold esterase according to the present invention allows to perform the hydrolysis of a compound of general formula (II) at high conversion and high selectivity.

In one embodiment, the compound of general formula (II) is hydrolysed if the α/β-hydrolase-fold esterase is a carboxyl esterase comprising or consisting of an amino acid sequence with at least 80 %, preferably at least 85%, more preferably at least 90 %, even more preferably at least 95 %, even more preferably at least 98%, even more preferably at least 99%, identity to SEQ ID No. 4, wherein said α/β-hydrolase-fold esterase comprises the catalytic motif of Ser-(Z)ₙ-Glu-(Z)ₘ-His (SEQ ID No. 5), wherein (Z)ₙ and (Z)ₘ are a chain of amino acids of a length of 20 to 200 amino acids. Preferably, the compound of general formula (II) is hydrolysed if the α/β-hydrolase-fold esterase is Esterase C from Rhodococcus rhodochrous (SEQ ID No. 4).

According to one embodiment of the inventive method, the composition described herein comprises the compound of general formula (II) and the compound of general formula (II'), wherein said composition is preferably a racemic composition, and the α/β-hydrolase-fold esterase is a carboxyl esterase comprising or consisting of an amino acid sequence with at least 80 %, preferably at least 85%, more preferably at least 90 %, even more preferably at least 95 %, even more preferably at least 98%, even more preferably at least 99%, identity to SEQ ID No. 4, wherein said enzyme comprises the catalytic motif of Ser-(Z)ₙ-Glu-(Z)ₘ-His (SEQ ID No. 5), wherein (Z)ₙ and (Z)ₘ are a chain of amino acids of a length of 20 to 200 amino acids. Preferably, said carboxyl esterase has an amino acid sequence of SEQ ID No. 4.

Preferably, the compound of general formula (II) is hydrolysed to the corresponding alcohol in said composition comprising a compound of general formula (II) and (II') in an enantiomeric excess of at least 80 %, preferably at least 85%, even more preferably at least 90 %.

Thus, the inventive method is also particularly advantageous to separate enantiomers of general formula (II) and (II') using an α/β-hydrolase-fold esterase which is a carboxyl esterase comprising or consisting of an amino acid sequence with at least 80 %, preferably at least 85%, more preferably at least 90 %, even more preferably at least 95 %, even more prefer-ably at least 98%, even more preferably at least 99%, identity to SEQ ID No. 4.

According to a preferred embodiment, the compound of general formula (II) is a (1S,2R,4S)-bornylester and said compound of general formula (II') is a (1R,2S,4R)-bornylester and said (1S,2R,4S)-bornylester is hydrolysed to (1S,2R,4S)-borneol. Preferably, said (1S,2R,4S)-bornylester is (1S,2R,4S)-bornylbutyrate and said (1R,2S,4R)-bornylester is (1R,2S,4R)-bornylbutyrate. It has been found that a particularly high conversion and selectivity is obtained if said (1S,2R,4S)-bornylester is (1S,2R,4S)-bornylbutyrate and said (1R,2S,4R)-bornylester is (1R,2S,4R)-bornylbutyrate.

Said (1S,2R,4S)-borneol may be further converted to (1S)-camphor. (1S)-camphor can be obtained by oxidizing (1S,2R,4S)-borneol, as described above.

The present invention further provides a composition comprising (1S,2S,4S)-isoborneol and (1R,2R,4R)-isobornylester obtainable by a method as described herein.

The present invention also provides a composition comprising (1S,2R,4S)-borneol and (1R,2S,4R)-bornylester obtainable by a method described herein.

The present invention is further illustrated by the following embodiments and examples, however, without being restricted thereto.

Preferred embodiments of the present invention:
1. A method for hydrolysing a compound of general formula (I) 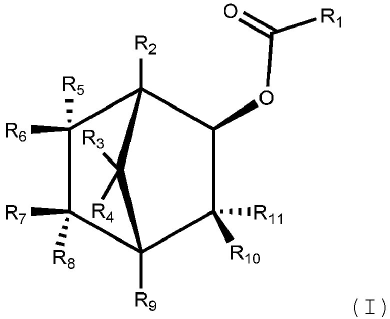 or a compound of general formula (II) 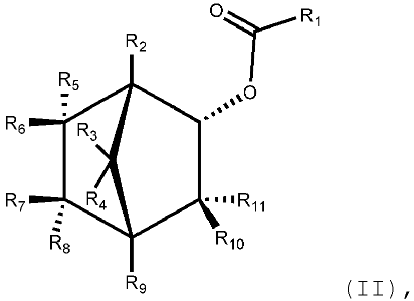 wherein R₁, R₂, R₃ and R₄ are independently from each other a C₁ to C₅ alkyl group or hydrogen, and R₅, R₆, R₇, R₈, R₉, R₁₀ and R₁₁ are independently from each other a C₁ or C₂ alkyl group or hydrogen,
   said method comprising the step of incubating at least one compound of general formula (I) or (II) with an α/β-hydrolase-fold esterase.
2. The method of embodiment 1, wherein the compound of general formula (I) or (II) is comprised in a composition further comprising a compound of general formula (I') 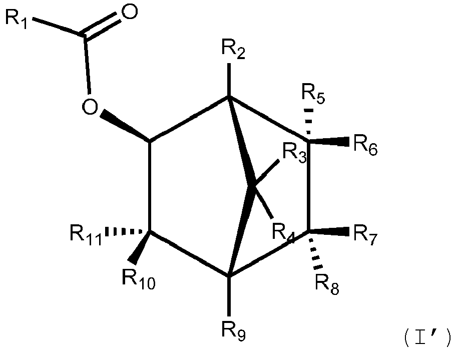 or a compound of general formula (II') 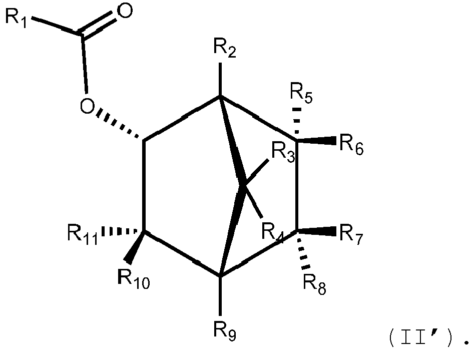
3. The method of embodiment 2, wherein said composition comprises the compound of general formula (I) and the compound of general formula (I'), or the compound of general formula (II) and the compound of general formula (II') .
4. The method of embodiment 3, wherein the composition is a racemic composition.
5. The method of any one of embodiments 1 to 4, wherein R1 is a methyl or propyl group.
6. The method of any one of embodiments 1 to 5, wherein R₂, R₃, R₄, R₁₀ and/or R₁₁ is a methyl group or hydrogen, and wherein R₅, R₆, R₇, R₈ and R₉ is hydrogen.
7. The method of any one of embodiments 1 to 6, wherein each of R₅, R₆, R₇, R₈, R₉, R₁₀ and R₁₁ is hydrogen.
8. The method of any one of embodiments 1 to 7, wherein each of R₂, R₃ and R₄ is a methyl group and wherein each of R₅, R₆, R₇, R₈, R₉, R₁₀ and R₁₁ is hydrogen.
9. The method of any one of embodiments 1 to 6, wherein each of R₂, R₁₀ and R₁₁ is a methyl group, and wherein each of R₃, R₄, R₅, R₆, R₇, R₈ and R₉ is hydrogen.
10. The method of any one of embodiments 1 to 9, wherein said α/β-hydrolase-fold esterase is a carboxyl esterase.
11. The method of any one of embodiments 1 to 10, wherein said α/β-hydrolase-fold esterase is a carboxyl esterase comprising or consisting of an amino acid sequence with at least 80 %, preferably 90 % identity to SEQ ID No. 1, wherein said α/β-hydrolase-fold esterase comprises the catalytic motif of Ser-X-X-Lys (SEQ ID No. 2), wherein X is any amino acid residue.
12. The method of embodiment 11, wherein said carboxyl esterase is wild type Esterase B from B. gladioli (SEQ ID No. 1) or the NK70 mutant thereof (SEQ ID No. 3).
13. The method of embodiment 11 or 12, wherein the compound of general formula (I) or (II) is hydrolysed.
14. The method of any one of embodiments 1 to 10, wherein said α/β-hydrolase-fold esterase is a carboxyl esterase comprising or consisting of an amino acid sequence with at least 80 %, preferably 90 %, identity to SEQ ID No. 4, wherein said α/β-hydrolase-fold esterase comprises the catalytic motif of Ser-(Z)ₙ-Glu-(Z)ₘ-His (SEQ ID No. 5), wherein (Z)ₙ and (Z)ₘ are a chain of amino acids of a length of 20 to 200 amino acids.
15. The method of embodiment 14, wherein the compound of general formula (II) is hydrolysed.
16. The method of any one of embodiments 2 to 13, wherein said composition comprises the compound of general formula (I) and the compound of general formula (I'), or said composition comprises the compound of general formula (II) and the compound of general formula (II'), wherein said composition is preferably a racemic composition, and wherein said α/β-hydrolase-fold esterase is a carboxyl esterase comprising or consisting of an amino acid sequence with at least 80 %, preferably 90 %, identity to SEQ ID No. 1, wherein said α/β-hydrolase-fold esterase comprises the catalytic motif of Ser-X-X-Lys (SEQ ID No. 2), wherein X is any amino acid residue.
17. The method of embodiment 16, wherein said carboxyl esterase has an amino acid sequence of SEQ ID No. 1 or SEQ ID No. 3.
18. The method of embodiment 16 or 17, wherein said compound of general formula (I) is a (1S,2S,4S)-isobornylester and said compound of general formula (I') is a (1R,2R,4R)-isobornylester and said (1S,2S,4S)-isobornylester is hydrolysed to (1S,2S,4S)-isoborneol.
19. The method of embodiment 18, wherein said (1S,2S,4S)-isobornylester is (1S,2S,4S)-isobornylbutyrate and said (1R,2R,4R)-isobornylester is (1R,2R,4R)-isobornylbutyrate.
20. The method of embodiment 18 or 19, wherein said (1S,2S,4S)-isoborneol is further converted to (1S)-camphor.
21. The method of embodiment 16 or 17, wherein said compound of general formula (II) is a (1R,2R,4S)-endofenchylester and said compound of general formula (II') is a (1S,2S,4R)-endofenchylester and said (1R,2R,4S)-endofenchylester is hydrolysed to (1R,2R,4S)-endofenchol.
22. The method of embodiment 21, wherein said (1R,2R,4S)-endofenchylester is (1R,2R,4S)-endofenchylbutyrate and said (1S,2S,4R)-endofenchylester is (1S,2S,4R)-endofenchylbutyrate.
23. The method of any one of embodiments 2 to 10, 14 or 15, wherein said composition comprises the compound of general formula (II) and the compound of general formula (II'), wherein said composition is preferably a racemic composition, and wherein said α/β-hydrolase-fold esterase is a carboxyl esterase comprising or consisting of an amino acid sequence with at least 80 %, preferably 90 %, identity to SEQ ID No. 4, wherein said enzyme comprises the catalytic motif of Ser-(Z)n-Glu-(Z)m-His (SEQ ID No. 5), wherein (Z)n and (Z)m are a chain of amino acids of a length of 20 to 200 amino acids.
24. The method of embodiment 23, wherein said carboxyl esterase has an amino acid sequence of SEQ ID No. 4.
25. The method of embodiment 22 or 23, wherein said compound of general formula (II) is a (1S,2R,4S)-bornylester and said compound of general formula (II') is a (1R,2S,4R)-bornylester and said (1S,2R,4S)-bornylester is hydrolysed to (1S,2R,4S)-borneol.
26. The method of embodiment 25, wherein said (1S,2R,4S)-bornylester is (1S,2R,4S)-bornylbutyrate and said (1R,2S,4R)-bornylester is (1R,2S,4R)-bornylbutyrate.
27. The method of embodiment 25 or 26, wherein said (1S,2R,4S)-borneol is further converted to (1S)-camphor.
28. The method of any one of embodiments 1 to 27, wherein the hydrolysis is performed at a pH of 4 to 9, preferably, 7 to 8, and at a temperature of 10 to 60 °C, preferably 20 to 30 °C.
29. A composition comprising (1S,2S,4S)-isoborneol and (1R,2R,4R)-isobornylester obtainable by a method according embodiment 18 or 19.
30. A composition comprising (1S,2R,4S)-borneol and (1R,2S,4R)-bornylester obtainable by a method according to embodiment 25 or 26.

### BRIEF DESCRIPTION OF THE FIGURES

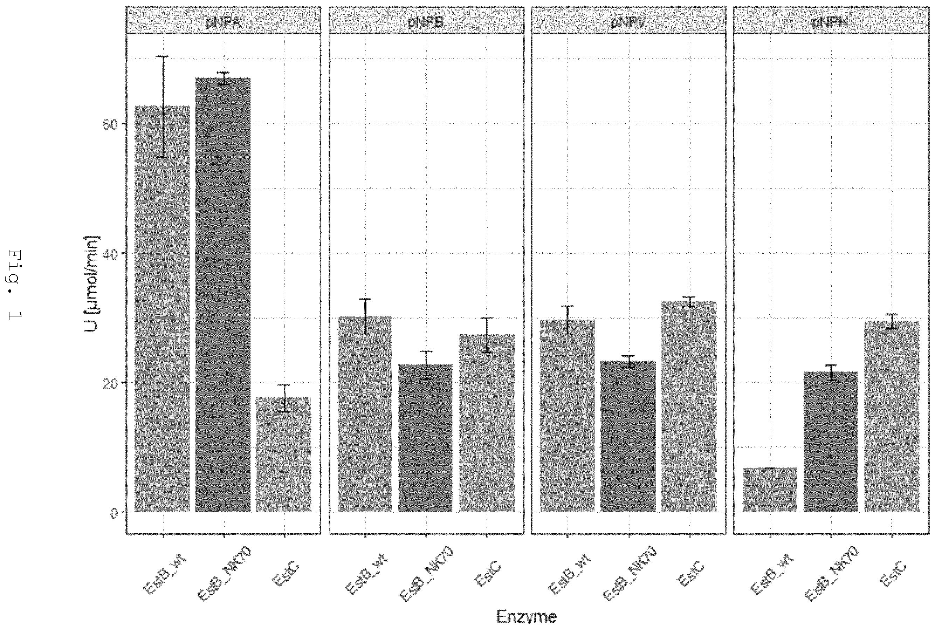

Fig. 1 shows activity measurements for the hydrolysis of esters by EstB and EstC. As model substrates, different p-nitrophenyl esters were used: acetate (pNPA), butyrate (pNPB), valerate (pNPV) and hexanoate (pNPH). All measurements were carried out in triplicate. A unit is defined as 1 µmol of p-nitrophenol produced per minute.

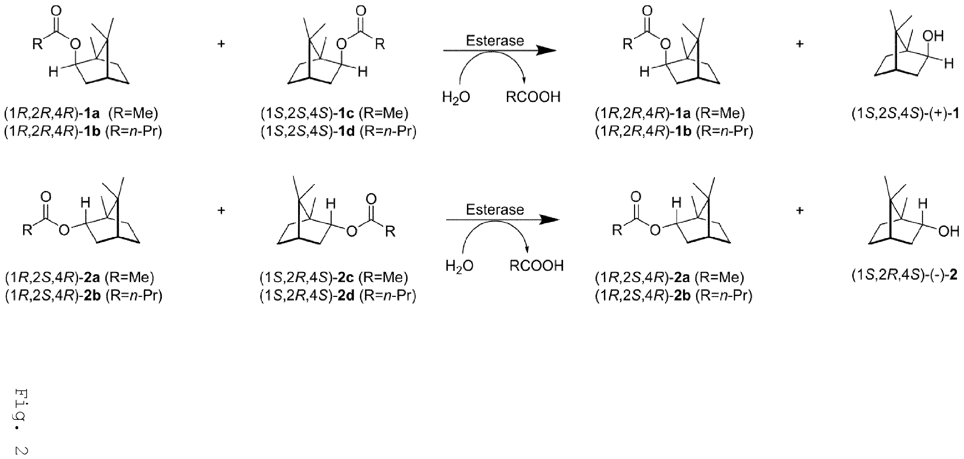

Fig. 2 shows a chemical reaction scheme of the kinetic resolution of acetyl and butyryl esters of isoborneol (1, above) and borneol (2, below). Compound 1a: (1R,2R,4R)-isobornylacetate; compound 1b: (1R,2R,4R)-isobornylbutyrate; compound 1c: (1S,2S,4S)-isobornylacetate, compound 1d: (1S,2S,4S)-isobornylbutyrate; compound 2a: (1R,2S,4R)-bornylacetate; compound 2b: (1R,2S,4R)-bornylbutyrate; compound 2c: (1S,2R,4S)-bornylacetate; compound 2d: (1S,2R,4S)-bornylbutyrate.

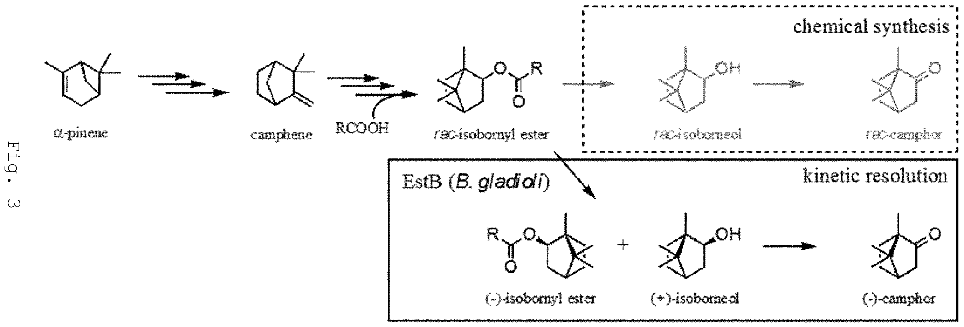

Fig. 3 shows a chemical reaction scheme for the synthesis of racemic camphor from α-pinene. Esterase-catalysed kinetic resolution of bornyl esters substitutes unselective ester hydrolysis and allows the isolation of both isomers in pure form.

### EXAMPLES

### Materials and Methods

### Materials:

All chemicals were purchased from either Sigma Aldrich Chemie (Steinheim, Germany), Alfa-aesar (Thermo Fisher (Kandel) GmbH, Germany), c-LEcta (Leipzig, Germany) unless otherwise stated.

### Enzymes:

Esterases were produced in terrific broth (TB) media at 20 °C overnight according to the standard protocol after adding the appropriate antibiotic and inducer listed in Table 1.

**Table 1. List of enzymes used in this work and organism of origin.**

| Enzyme | Organism | Plasmid / Antibiotic / Inducer |
|---|---|---|
| Esterase | Porcine liver | Immobilised (Commercial) |
| Esterase EstB | *Burkholderia gladioli* | pK214_estB (wt) / kan /IPTG |
| Esterase EstA | *Burkholderia gladioli* | pJexpress401_estA / kan / ARA |
| Esterase EstA | *Rhodococcus rhodochrous* | pMS470_estA /amp / IPTG |
| Esterase EstC | *Rhodococcus rhodochrous* | pMS470d8-estC /amp / IPTG |
| Esterase EstA | *Arthrobacter nicotianae* | pMS470_estA /amp / IPTG |
| Esterase EstB mutant a | *Burkholderia gladioli* | pK214_estB (NK70) / kan /IPTG |

| | | |
|---|---|---|
| ^{a} Mutations confirmed by sequencing: S17L G132S E251G A311V E316K | | |

### Synthesis of monoterpenol esters:

**Bornyl and isobornyl esters:** To 1 g of either borneol or isoborneol (50 mM) and dimethylaminopyridine (DMAP, 6 eq.) in dry dichloromethane (130 mL, respectively), acetyl, butyryl or hexanoyl chloride (4 eq.) was added dropwise and the solution was stirred overnight. The mixture was washed with 1M HCl (2x 60 mL) and distilled water (2x 60 mL). The organic layer was dried over anhydrous Na2SO4 before the solvent was removed under reduced pressure. Products were purified with column chromatography (cyclohexane:ethyl acetate, 95:5 for the acetyl esters and 90:10 for the butyryl esters) and isolated yields of 70-85% were reached. Bornyl acetate, bornyl butyrate, isobornyl butyrate, isobornyl hexanoate and endofenchyl butyrate were identified based on the 1H-NMR-spectra from literature.

**Endofenchyl butyrate:** To 100 mg of endofenchol and dimethylaminopyridine (DMAP, 6 eq.) in dry dichloromethane (13 mL, respectively), butyryl chloride (4 eq.) was added dropwise and the solution was stirred overnight. The mixture was washed with 1M HCl (2 x 6 mL) and distilled water (2x 6 mL). The organic layer was dried over anhydrous Na2SO4 before the solvent was removed under reduced pressure. Product was purified with column chromatography (cyclohexane:ethyl acetate, 97:3) and isolated yield of 70% was reached. Endofenchyl butyrate was identified based on the 1H-NMR-spectra from literature.

### Conversion of optically pure borneol enantiomers using lipases

All biotransformations were carried out in 1 mL anhydrous tert-butylmethylether (tBME) using 5 mM of either (+)- or (-)-borneol, 3 equivalent of vinyl butyrate (15 mM) and 4A molecular sieve. 10 mg/mL or 50 mg/mL of each immobilized commercial lipase listed in

Table 1 was added and the reactions were carried out at 30 °C. 300 µl samples were periodically taken, dried over sodium sulphate and submitted to GC-FID analysis to conversion.

### Enzymatic kinetic resolution with esterases

All kinetic resolutions were carried out using 1 mM of either bornyl, isobornyl or endofenchyl esters in 50 mM potassium phosphate buffer pH 7 at 30°C for 24h with stirring at 600 rpm. All esterases described in

Table 1 were applied as cell-free extract (9 g/L total protein content). All reactions were carried out in duplicate, samples of 200 µl were periodically taken, extracted with 200 µl of ethyl acetate, dried over sodium sulphate and submitted to GC-FID analysis.

### p-nitrophenyl ester assay

In a microtiter plate, of cleared cell lysate (10 µL), either directly after cell disruption or after 10-fold dilution, were added to Tris-HCl buffer (180 µL 100 mM Tris-HCl, 150 mM NaCl, pH 7.5). Absorbance over time (λ = 410 nm) was measured in the spectrophotometer (BioTek, USA) immediately after the addition of 10 µL of 20 mM DMSO stock of one of the p-nitrophenyl esters: acetate, butyrate, valerate or hexanoate. The esterases activity in CFE was calculated based on the calibration as the initial reaction velocity (µmol/min). The extinction coefficient calculated for p-nitrophenolate with the calibration curve at the stated pH and buffer was 17700 M/cm.

### Results

### Kinetic resolution using esterases

In a prescreen of a collection of 15 bacterial esterases in the conversion of sterically demanding esters, five had shown activity towards monoterpenes such as 5-endo-norbonen-2-yl acetate or tetrahydrofuran-3-acetate (data not shown). As 5-endo-norbonen-2-yl acetate shares the same bicyclic structure as esters from isoborneol and borneol, the five esterases that converted this substrate were tested in the conversion of the α-acetate or butyrate esters of isoborneol and borneol (Table 2). In a pre-screen by thin-layer chromatography, two out of five of the tested esterases, EstB from Burkholderia gladioli and EstC from Rhodococcus rhodochrous (SEQ. ID No. 4), showed activity in the hydrolysis of bornyl and isobornyl acetate, respectively. For EstB, the wild-type enzyme (SEQ ID No. 1) and mutant EstB NK70 (SEQ ID No.3) were investigated.

This variant with seven amino acid substitutions was generated by directed evolution and contains the mutation A311V among others which is believed to stabilize a relatively unstable loop. EstB NK70 has increased temperature stability (with a 13°C higher Tₘ and a 50-fold longer half-life time at 50°C) and higher tolerance against water-soluble co-solvents, which was considered advantageous in view of a later implementation of the kinetic resolution. After the identification of active enzymes, w the preference of the enzymes towards p-nitrophenol esters of different lengths (C₂, C₄, C₅, C₆) was determined. This revealed a clear preference of EstB and EstB NK70 towards shorter esters whereas EstC seems to prefer longer ones (Fig. 1).

In the kinetic resolution of racemic esters of isoborneol and borneol, all esterases except the commercial esterase from Porcine liver showed better selectivity with butyrate substituent compare to the shorter counterpart, resulting in up to 7-fold higher E values towards the butyrates. Furthermore, both EstB variants showed particularly high enantioselectivity in the kinetic resolution of isobornyl butyrate, whereas EstC showed high selectivity for bornyl butyrate (Table 2). Interestingly, EstB converted all substrates, EstC, in contrast, showed no conversion with isobornyl esters as substrate. Conversion of isobornyl hexanoate by EstB, its variant NK70 and EstC yielded no product (data not shown), which was surprising since the corresponding p-nitrophenyl ester was converted, albeit with much lower activity than esters of shorter acids.

**Table 2. Kinetic resolution of bornyl butyrate and isobornyl butyrate using 1 mM substrate, data reported after 24 h reaction time.**

| Substrate | Enzyme | Origin | Conversion¹ [%] | ee_{P}¹ [%] | E- value² | preferentially formed product enantiomer |
|---|---|---|---|---|---|---|
| rac-bornyl acetate | Esterase | Porcine liver | 49.4% ± 4.3% | 0.3 | 1 | n.d. |
| *rac*-bornyl butyrate | Esterase | Porcine liver | 59.4% ± 1.2% | 0.1 | 1 | n.d. |
| *rac*-isobornyl acetate | Esterase | Porcine liver | 43.7% ± 0.1% | 80% | 17 | (-)-isoborneol |
| *rac-* isobornyl butyrate | Esterase | Porcine liver | 42.6% ±1.7% | 21% | 2 | (-)-isoborneol |
| *rac*-isobornyl acetate | EstB wt | *B. gladioli* | 16.90% | 91% | 26 | (+)-isoborneol |
| *rac-* isobornyl butyrate | EstB wt | *B. gladioli* | 31.2% ± 0.1% | 98% | >100 | (+)-(+)-isoborneol |
| *rac-*endofenchyl butyrate | EstB wt | *B. gladioli* | 35% ±2.0% | 99% | >100 | (+)-endofenchol |
| *rac*-bornyl acetate | EstB NK70 | *B. gladioli* | 24.8% ±2.0% | 31% | 2 | (+)-borneol |
| *rac*-bornyl butyrate | EstB NK70 | *B. gladioli* | 65.5% ±1.2% | 37% | 4 | (+)-borneol |
| *rac*-isobornyl acetate | EstB NK70 | *B. gladioli* | 23.2% ± 0.1% | 91% | 30 | (+)-(+)-isoborneol |
| *rac-* isobornyl butyrate | EstB NK70 | *B. gladioli* | 31.2%± 0% | 98% | >100 | (+)-isoborneol |
| *rac*-bornyl acetate | EstC | *R. rhodochrous* | 11.2% ±1.5% | 89% | 18 | (-)-borneol |
| *rac*-bornyl butyrate | EstC | *R. rhodochrous* | 31.8% ± 0.1% | 97% | >100 | (-)-borneol |
| *rac*-isobornyl acetate | EstC | *R*. *rhodochrous* | n.c. | n.c. | n.c. | n.c. |
| *rac-* isobornyl butyrate | EstC | *R. rhodochrous* | n.c. | n.c. | n.c. | n.c. |

| | | | | | | |
|---|---|---|---|---|---|---|
| ¹Determined by chiral gas chromatography; ²calculated from conversion and ee_{P} according to Chen et al., J. Am. Chem. Soc. 1982, 104, 7294-7299. n.d.: not determined ; n.c.: no conversion | | | | | | |

Esterase B from Burkholderia gladioli is known to convert esters of bulky alcohols (such as linalyl acetate), albeit often with very low selectivity. Surprisingly, EstB from B. gladioli showed high activity and excellent enantioselectivity in the kinetic resolution of rac-isobornyl butyrate giving rise to (+)-isoborneol (or (1S,2S,4S)-1,7,7-Trimethyl-bicyclo[2.2.1]heptan-2-ol in high optical purity (>98% ee). Hydrolysis of rac-isobornyl acetate produced (+)-isoborneol in 89% ee. The oxidation of (+)-isoborneol is simple and gives rise to (-)-camphor (or (1S)-camphor). In contrast, EstC showed high enantioselectivity towards rac-bornyl butyrate and produced (-)-borneol in 97% ee. From rac-bornyl acetate, (-)-borneol was produced with 90% ee.

Fig. 2 shows a chemical scheme of the kinetic resolution of acetyl and butyryl esters of isoborneol and borneol.

EstB is a hydrolase that exhibits homology with esterases of class VIII and with class C β-lactamases. The natural substrate is presumably Cephalosporin C, a carboxyl amide. While the enzyme shares the same fold with frequently used esterases and lipases, its catalytic machinery differs considerable. This regards the position of the catalytic serine 105 and the nature of the catalytic triad, which does not consist of the typical motif Ser-His-Asp/Glu, but of Ser75, Lys78 and/or Trp348. While the enzyme has been shown to accept sterically very demanding tertiary alcohols, the selectivity towards all racemic substrates investigated has been low so far.

It was a surprising finding that the Cephalosporin esterase EstB has high selectivity towards racemic isobornyl esters. This selectivity can be attributed to the difference in free enthalpy of the transitions states leading to and from the first tetrahedral intermediate in the mechanism, which in turn depends on differences between the two substrate enantiomers (covalently bound to the catalytic serine) in their molecular interactions with amino acids in the active site pocket of EstB.

It was another surprising finding that the Cephalosporin esterase EstB has high selectivity towards racemic endofenchyl esters.

Interestingly, EstB show inverse enantiopreference towards esters from borneol and isoborneol as it preferentially produced (+)-borneol (whose oxidation leads to (+)-camphor) from racemic bornyl butyrate, whereas it produces (+)-isoborneol from racemic isobornyl butyrate (whose oxidation leads to (-)-camphor). The different endo-and exo-position of the ester group in α/β-position of the bicyclic molecules leads to an inversion of the preference of the enzyme.

Implementation of enzyme catalysis can either replace one or more existing steps of a given synthetic route, or a new synthetic route is assembled "de novo". The latter is typically hard to implement as it requires a complete redesign of a certain chemical process, whereas replacing only one step is considerably easier. While the yield limitation of kinetic resolutions is considered a disadvantage, it is still very popular in industrial processes due to the extremely high simplicity. The EstB-catalysed kinetic resolution process can be easily integrated into an existing chemical route (Fig. 3). Fig. 3 shows the synthesis of racemic camphor from α-pinene. Esterase-catalysed kinetic resolution of bornyl esters can substitute unselective ester hydrolysis and allows the isolation of both isomers in pure form.

Isobornyl and bornyl esters are both used as fragrances and in food preparations and as chiral ligands, or they can be hydrolysed and oxidized to produce the natural (+)-camphor and (-)-camphor isomers. The biocatalytical step simply substitutes the unselective chemical hydrolysis (see Fig. 3). Unlike oxidoreductases, the esterase does not require supply of external cofactors or additional steps for their regeneration. This invention underlines the simplicity of this approach where the production of the highly valuable (1S)-camphor using an enzyme can outweigh the cost of a chromatographic separation of enantiomers. Racemic borneol and bornyl acetate as starting material for EstC are not readily available but are common compounds in turpentine from different trees and can be isolated during the refinement.

The cheap isobornyl butyrate can be used as substrate for EstB from B. gladioli followed by the further oxidation of (+)-isoborneol to the highly valuable (-)-camphor. In contrast, EstC from R. rhodochrous can catalyse the kinetic resolution of the bornyl esters affording (-)-borneol which can be oxidised to the valuable (-)-camphor as well.

Turpentine oils, a waste by-product from industrial wood processing were identified as possible substituents for fossil derived chemicals. The identification of stereoselective enzymes for the synthesis of high value compounds from bio-based side-stream can substitute petrol chemicals and the isolation of natural product from plants (which is problematic due to considerable the accumulation of waste), respectively, and makes thus a contribution towards the development of clean and economically successful processes.

## Claims

1. A method for hydrolysing a compound of general formula (I) 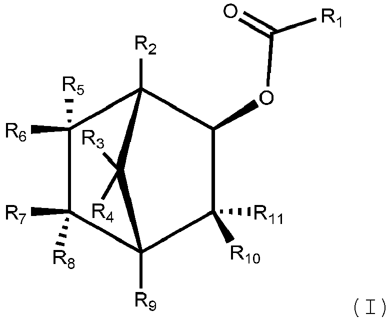 or a compound of general formula (II) 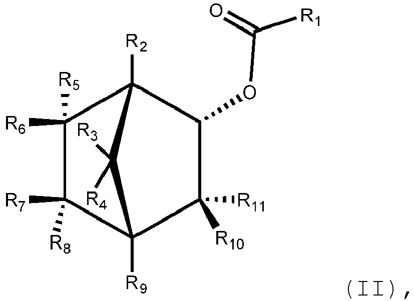 wherein R₁, R₂, R₃ and R₄ are independently from each other a C₁ to C₅ alkyl group or hydrogen, and R₅, R₆, R₇, R₈, R₉, R₁₀ and R₁₁ are independently from each other a C₁ or C₂ alkyl group or hydrogen,
said method comprising the step of incubating at least one compound of general formula (I) or (II) with an α/β-hydrolase-fold esterase.

2. The method of claim 1, wherein the compound of general formula (I) or (II) is comprised in a composition further comprising a compound of general formula (I') 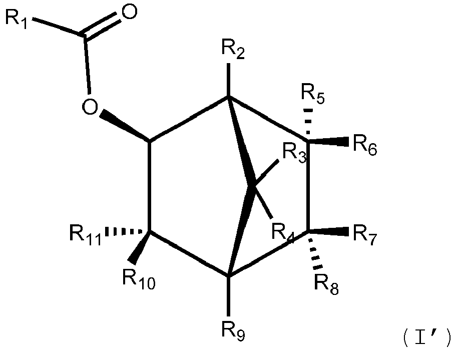 or a compound of general formula (II')

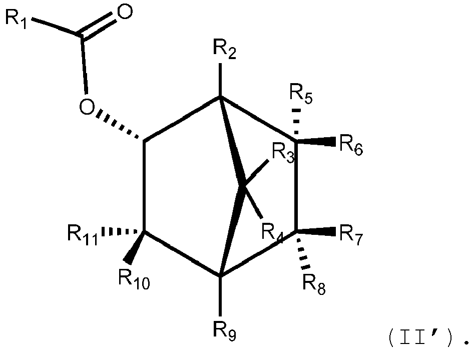

3. The method of claim 2, wherein said composition comprises the compound of general formula (I) and the compound of general formula (I'), or the compound of general formula (II) and the compound of general formula (II').

4. The method of any one of claims 1 to 3, wherein R₁ is a methyl or propyl group.

5. The method of any one of claims 1 to 4, wherein R₂, R₃, R₄, R₁₀ and/or R₁₁ is a methyl group or hydrogen, and wherein R₅, R₆, R₇, R₈ and R₉ is hydrogen.

6. The method of any one of claims 1 to 5, wherein said α/β-hydrolase-fold esterase is a carboxyl esterase.

7. The method of any one of claims 1 to 6, wherein said α/β-hydrolase-fold esterase is a carboxyl esterase comprising or consisting of an amino acid sequence with at least 80%, preferably 90% identity to SEQ ID No. 1, wherein said α/β-hydrolase-fold esterase comprises the catalytic motif of Ser-X-X-Lys (SEQ ID No. 2), wherein X is any amino acid residue.

8. The method of any one of claims 1 to 6, wherein said α/β-hydrolase-fold esterase is a carboxyl esterase comprising or consisting of an amino acid sequence with at least 80%, preferably 90%, identity to SEQ ID No. 4, wherein said α/β-hydrolase-fold esterase comprises the catalytic motif of Ser-(Z)ₙ-Glu-(Z)ₘ-His (SEQ ID No. 5), wherein (Z)ₙ and (Z)ₘ are a chain of amino acids of a length of 20 to 200 amino acids.

9. The method of claim 8, wherein the compound of general formula (II) is hydrolysed.

10. The method of any one of claims 2 to 7, wherein said composition comprises the compound of general formula (I) and the compound of general formula (I'), or said composition comprises the compound of general formula (II) and the compound of general formula (II'), wherein said composition is preferably a racemic composition, and wherein said α/β-hydrolase-fold esterase is a carboxyl esterase comprising or consisting of an amino acid sequence with at least 80%, preferably 90%, identity to SEQ ID No. 1, wherein said α/β-hydrolase-fold esterase comprises the catalytic motif of Ser-X-X-Lys (SEQ ID No. 2), wherein X is any amino acid residue.

11. The method of claim 10, wherein said compound of general formula (I) is a (1*S*,2*S*,4*S*)-isobornylester and said compound of general formula (I') is a (1*R*, 2*R*, 4*R*) - isobornylester and said (1S,2S,4S)-isobornylester is hydrolysed to (1*S,* 2*S,* 4*S*)-isoborneol.

12. The method of claim 11, wherein said (1S,2S,4S)-isoborneol is further converted to (1S)-camphor.

13. The method of claim 10, wherein said compound of general formula (II) is a (1R,2R,4S)-endofenchylester and said compound of general formula (II') is a (1*S*,2*S*,4*R*)-endofenchylester and said (1*R*,2*R*,4*S*)-endofenchylester is hydrolysed to (1*R*,2*R*,4*S*)-endofenchol.

14. The method of any one of claims 2 to 6, 8 or 9, wherein said composition comprises the compound of general formula (II) and the compound of general formula (II'), wherein said composition is preferably a racemic composition, and wherein said α/β-hydrolase-fold esterase is a carboxyl esterase comprising or consisting of an amino acid sequence with at least 80 %, preferably 90 %, identity to SEQ ID No. 4, wherein said enzyme comprises the catalytic motif of Ser-(Z)ₙ-Glu-(Z)ₘ-His (SEQ ID No. 5), wherein (Z)ₙ and (Z)ₘ are a chain of amino acids of a length of 20 to 200 amino acids.

15. The method of claim 14, wherein said compound of general formula (II) is a (1S,2R,4S)-bornylester and said compound of general formula (II') is a (1R,2S,4R)-bornylester and said (1S,2R,4S)-bornylester is hydrolysed to (1S,2R,4S)-borneol.

16. A composition comprising (1S,2S,4S)-isoborneol and (1R,2R,4R)-isobornylester obtainable by a method according claim 11.

17. A composition comprising (1S,2R,4S)-borneol and (1*R*,2*S*,4*R*)-bornylester obtainable by a method according to claim 15.
